# EUROPÄISCHE PATENTSCHRIFT

(11) **EP 2 628 470 B1**
(45) Veröffentlichungstag und Bekanntmachung des Hinweises auf die Patenterteilung: **25.11.2020**
(21) Anmeldenummer: 12196723.6
(22) Anmeldetag: 12.12.2012
(51) Int. Cl.: A61F 2/966

(54) **Freigabevorrichtung zum Lösen eines medizinischen Implantats von einem Katheter und Katheter mit einer Freigabevorrichtung sowie Verfahren zum Klemmen eines Implantats in einem solchen**
Release device for releasing a medical implant from a catheter and catheter having a release device and method for clamping an implant therein
Dispositif de libération destiné à libérer un implant médical d'un cathéter et cathéter avec un dispositif de libération ainsi que procédé de serrage d'un implant dans celui-ci

(30) Priorität: 16.02.2012 US 201261599430 P
(43) Veröffentlichungstag der Anmeldung: 21.08.2013
(73) Patentinhaber: Biotronik AG, 8180 Bülach (CH)
(72) Erfinder: Fargahi, Amir, 8180 Bülach (CH)
(74) Vertreter: Randoll, Sören

(56) Entgegenhaltungen:
- EP-A1- 1 369 098
- WO-A1-2011/066961
- WO-A2-2011/014814
- DE-A1-102009 020 012
- US-A- 5 709 703
- US-A1- 2007 233 224
- US-A1- 2009 287 290
- US-A1- 2009 287 292
- US-B1- 6 858 034

## Beschreibung

Die Erfindung betrifft eine Freigabevorrichtung zum Lösen eines medizinischen Implantats von einem Katheter und einen Katheter mit einer Freigabevorrichtung zum Freigeben eines medizinischen Implantats zur Implantation in einen tierischen und/oder menschlichen Körper sowie ein Verfahren zum Klemmen eines Implantats in einem solchen Katheter nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate werden vor einem Einführen in den Körper mit Kathetern verbunden und müssen so befestigt sein, dass sie am Einsatzort komplikationslos vom Katheter genau platziert und definiert freigegeben werden können. Hierzu ist beispielsweise bekannt, das Implantat mit Ösen auszustatten, die mit Haken am Katheter interagieren und so das Implantat am Katheter befestigen.

WO 2011/066961 A1 und WO 2011/014914 A2 offenbaren einige Freigabevorrichtungen. US5709703 offenbart eine Freigabevorrichtung nach dem einleitenden Teil des Anspruchs 1.

Der Erfindung liegt die Aufgabe zugrunde, eine Freigabevorrichtung anzugeben, mit der ein Verbinden eines Implantats mit einer Einführvorrichtung einfach und bedienerfreundlich möglich ist und mit der ein hochpräzises und gezieltes Freigeben eines Implantats erfolgen kann.

Eine weitere Aufgabe ist in der Bereitstellung einer entsprechenden Einführvorrichtung zu sehen.

Zudem ergibt sich eine weitere Aufgabe in der Bereitstellung eines Verfahrens zum Klemmen eines Implantats mittels einer entsprechenden Freigabevorrichtung in einer Einführvorrichtung.

Die Aufgabe wird erfindungsgemäß durch die Merkmale der unabhängigen Ansprüche 1 und 16 gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst eine Klemmeinheit zum Klemmen des Implantats in der Einführvorrichtung mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei die Klemmeinheit ein erstes Bauteil und zumindest ein zweites Bauteil umfasst, die das Implantat im Klemmzustand zwischen sich einklemmen.

Durch die erfindungsgemäße Ausgestaltung kann eine Freigabevorrichtung bereitgestellt werden, in der das Implantat sicher gehalten wird bzw. auf einem Einführelement, wie beispielsweise einem Innenschaft, der Einführvorrichtung sicher befestigt ist. Die Klemmeinheit erlaubt ferner eine kompakte und einfache Konstruktion der Freigabevorrichtung. Zudem weist die Freigabevorrichtung eine einfache Handhabung und Montage des Implantats auf der Einführvorrichtung, beispielsweise einem Katheter, im Vorbereitungslabor auf. Des Weiteren ist eine Freigabe des Implantats zuverlässig und schnell. Ferner kann ein Risiko einer Deformation des Implantats sowie einer daraus resultierende Blockade der Freigabevorrichtung, wie sie bei Vorrichtungen des Standes der Technik, die mit Haken und Ösen arbeiten, auftreten, eliminiert werden.

In diesem Zusammenhang soll unter einer "Klemmeinheit" eine Einheit verstanden werden, die mittels einer Klemmwirkung und/oder eines Kraftschlusses ein anderes Element, insbesondere das Implantat, an einem Bauteil der Einführvorrichtung, insbesondere einem Einführelement bzw. dem Innenschaft, in einer festgelegten Position hält. Hierbei kann die Klemmeinheit die Klemmwirkung selbst vermitteln oder die Klemmeinheit und/oder das erste und/oder das zweite Bauteil können mit zumindest einem weiteren Element, insbesondere der Einführvorrichtung, wie beispielsweise einem Einführelement bzw. dem Innenschaft und/oder einem Außenschaft der Einführvorrichtung, zusammenwirken. Zusätzlich kann die Klemmeinheit eine von der Klemmwirkung separat ausgelegte Haltewirkung, beispielsweise durch eine Materialeigenschaft, aufweisen. Ein "Klemmzustand" stellt hier einen Zustand dar in dem das Implantat verliersicher in der Einführvorrichtung gehalten wird. Das erste und das zweite Bauteil üben einen Klemmsitz auf das Implantat aus. Ferner können das erste und das zweite Bauteil baugleich oder verschieden ausgeführt sein. Zudem ist das erste und/oder das zweite Bauteil kein Bestandteil des Implantats oder nicht am Implantat angeformt und/oder mit diesem stoffschlüssig verbunden. Bevorzugt klemmen die Bauteile das Implantat longitudinal und/oder transversal zwischen sich ein.

Vorteilhafterweise hält im Klemmzustand ein Formschluss zwischen dem ersten Bauteil und dem Implantat dieses in Position, wodurch ein Entgleiten des Implantats aus der Freigabevorrichtung bzw. des ersten Bauteils verhindert wird. Des Weiteren kann das Implantat zudem durch eine hohe Reibung eines Materials des ersten Bauteils verliersicher gehalten werden. Ferner ist es vorteilhaft, wenn eine Reibung zwischen dem zumindest zweiten Bauteil und dem Implantat dieses in Position hält. Hierdurch kann auf spezielle Befestigungselemente an der Klemmeinheit und/oder am Implantat, wie aus dem Stand der Technik bekannte Haken und Ösen, Bauraum sparend verzichtet werden. Diese Wechselwirkung von zweitem Bauteil und Implantat wird bevorzugt unterstützt durch eine Interaktion mit zumindest einem der Einführelement und insbesondere dem Außenschaft, wodurch das Implantat sicher gehalten wird.

Bevorzugt weist das erste Bauteil einen Hohlraum zur Aufnahme zumindest eines Teils des Implantats auf, wodurch eine Wechselwirkung zwischen dem ersten Bauteil und dem Implantat einfach erfolgen kann. Des Weiteren wird vorgeschlagen, dass das erste Bauteil zumindest einen Hüllbereich aufweist, wodurch das zweite Bauteil konstruktiv einfach den Formschluss mit dem Implantat eingehen kann. Unter einem "Hüllbereich" soll hier ein Anteil des ersten Bauteils verstanden werden, der sich im Klemmzustand zumindest über einen Bereich eines Umfangs des Implantats erstreckt und/oder dieses zumindest teilweise umschließt. Der Hüllbereich des ersten Bauteils kann beispielsweise ein Zylinder sein. Es wäre auch denkbar, dass das erste Bauteil von einem Zylinder gebildet ist. Im montierten Zustand des ersten Bauteils in der Einführvorrichtung ist der Hüllbereich bevorzugt am distalen Ende angeordnet. In einer vorteilhaften Ausgestaltung wird vorgeschlagen, dass im Klemmzustand der Hüllbereich des ersten Bauteils zumindest ein proximales Ende des Implantats umschließt, wodurch das erste Bauteil hin zum Anwender bzw. zum proximalen Ende der Einführvorrichtung angeordnet ist. Vorteilhafterweise ist am proximalen Ende des ersten Bauteils eine sich in eine radiale Richtung erstreckende Wandung angeordnet oder bevorzugt angeformt, die beispielsweise eine Angriffsfläche für eine Schiebebewegung des Implantats in der Einführvorrichtung bietet. Des Weiteren kann auch ein proximales Ende des ersten Bauteils einen Stopper aufweisen, der zur Limitierung einer Bewegung des Implantats in Richtung eines proximalen Endes der Einführvorrichtung, die zu einer Blockade des Implantats führen würde, dient.

In einer bevorzugten Realisierung ist das erste Bauteil von einer Buchse gebildet, wodurch die vorteilhaften Merkmale des Zylinders, der Wandung und des Stoppers in einem Bauteil vereint werden können. Das zumindest zweite Bauteil weist bevorzugt eine zylinderförmige Kontur auf und ist insbesondere im Wesentlichen als ein massiver Zylinder ausgebildet, wobei unter im Wesentlichen zu verstehen ist, dass mehr als 50% des Zylinders, bevorzugt mehr 65% und besonders bevorzugt mehr als 75% massiv ausgeführt sind. Durch die massive Ausgestaltung kann ein besonders stabiles Bauteil bereitgestellt werden. Ferner wird vorgeschlagen, dass das zumindest zweite Bauteil von einem Stecker gebildet ist, wodurch eine Wechselwirkung mit anderen Bauteilen konstruktiv einfach gewährleistet werden kann. Hierbei soll unter einem "Stecker" ein Bauteil verstanden werden, das in einem anderen Bauteil, wie einem Zylinder und/oder einer Buchse, angeordnet werden kann.

In einer vorteilhaften Realisierung weist das zumindest zweite Bauteil ein Material mit hoher Haftreibung auf, um das Implantat in dem Klemmzustand in Position zu halten, wodurch eine Fixierung des Implantats konstruktiv einfach erfolgen kann. Hierbei wird die Haftreibung zwischen dem zumindest zweiten Bauteil und einem Bereich des Implantats, insbesondere unmittelbar, in Richtung des distalen Endes, am proximalen Ende, in der Einführvorrichtung erzeugt. Das Material kann jedes, vom Fachmann für sinnvoll erachtetes Material sein, wie beispielsweise ein Polymer und insbesondere ein Material ausgewählt aus der Gruppe bestehend aus Polyamid, Polyester, Polyether-Block-Amid, Silikon, Polyurethan. Hierdurch kann das zumindest zweite Bauteil insbesondere mit geringem Gewicht ausgeführt werden. Eine besonders zuverlässige Positionierung des Implantats in der Einführvorrichtung kann wegen seiner hohen Haftreibung vorteilhaft erreicht werden, wenn das Material z.B. ein Polyether-Block-Amid wie PEBAX der Firma Arkema ist. Hierbei sind alle Härtegrade einsetzbar. Grundsätzlich kann das Material des zumindest zweiten Bauteils auch elastische und/oder federnde Eigenschaften haben. Es wäre auch möglich, dass das zumindest zweite Bauteil zur Erzeugung einer Klemmwirkung zumindest ein federndes Element, wie eine Druckfeder, aufweist.

Erfindungsgemäss wird vorgeschlagen, dass das erste Bauteil ein Material aufweist, das härter ist als das Material des zumindest zweiten Bauteils, wodurch das erste Bauteil besonders gut hohen Kräften, insbesondere Radialkräften des zweiten Bauteils und/oder des Implantats stand halten kann. Bevorzugt ist das Material insbesondere ein Material ausgewählt aus der Gruppe bestehend aus einem Kunststoff wie Polycarbonat, Polyetheretherketon PEEK, Perfluor-Ethylen-Propylen, high density Polyethylen, Polytetrafluorethylen, Polyamid, - bevorzugt einem harten Kunststoff, insbesondere Polycarbonat, Polyetheretherketon PEEK, Polyamid, - ein Metall, eine Keramik, ein Hart-Gummi oder ein Glas. Vorteilhafterweise ist das Material des ersten Bauteils ein Metall, bevorzugt ein rostfreier Stahl, wodurch ein gegenüber aggressiven Medien, wie beispielsweise Körperflüssigkeiten, resistentes und verträgliches Material eingesetzt werden kann.
In einer bevorzugten Realisierung sind das erste Bauteil und/oder das zumindest zweite Bauteil mit einem Monolayer-Design ausgeführt. Hierfür können für das erste Bauteil insbesondere Materialien wie ein Kunststoff (z. B. Polycarbonat, Polyetheretherketon PEEK, Polyamid) oder ein Metall (Stahl) eingesetzt werden und für das zumindest zweite Bauteil insbesondere Materialien wie Polyurethane, Silikone, Gummi, Polyamide, Polyester oder Polyether-Block-Amide, wie bevorzugt PEBAX.

In einer alternativen Ausgestaltung sind das erste Bauteil und/oder das zumindest zweite Bauteil mit einem Multilayer-Design bzw. mit einem so genannten Multilayer-Co-extrudiertes Design ausgeführt. Hierbei weist das erste Bauteil und/oder das zumindest zweite Bauteil zumindest zwei Schichten auf, die in radialer Richtung hintereinander bzw. übereinander angeordnet sind. Bei dem ersten Bauteil wird beispielsweise für eine radial äußere Schicht bzw. eine Außenschicht ein Material mit geringer Reibung ausgewählt und eine radial innere Schicht bzw. eine Innenschicht aus einem Material mit höherer Reibung. Hierdurch kann zum einen das erste Bauteil fast reibungslos in der Einführvorrichtung bzw. dessen Außenschaft bewegt werden und zum anderen das Implantat mit hoher Haltekraft im ersten Bauteil fixiert werden. Eine radial innere Schicht bzw. eine Innenschicht des zumindest zweiten Bauteils ist bevorzugt aus einem Material mit geringer Reibung ausgewählt und eine radial äußere Schicht bzw. eine Außenschicht aus einem Material mit höherer Reibung. Dadurch kann das zumindest zweite Bauteil beispielsweise auf dem Innenschaft der Einführvorrichtung leicht bewegt werden und gleichzeitig kann die hohe Reibung zwischen der Außenschicht des zumindest zweiten Bauteils und dem Implantat realisiert werden.

Die Schicht mit geringer Reibung weist hierbei bevorzugt ein Material ausgewählt aus der Gruppe bestehend aus Perfluor-Ethylen-Propylen (FEP), high density Polyethylen (HDPE), Polytetrafluorethylen (PTFE, Teflon) oder hydrophob-/hydrophil-beschichtete Polyamide (PA), insbesondere PA-6, -6.6, -6.10, -6.12, -11, -12, auf. Die Schicht mit höherer Reibung weist hierbei bevorzugt ein Material ausgewählt aus der Gruppe bestehend aus Polyamid, Polyester, Polyether-Block-Amid (PEBAX), Silikon, Polyurethan, (PUR), Gummi auf. Zu einer guten Kontaktierung der zwei Schichten kann eine weitere, mittlere Schicht vorgesehen sein. Diese mittlere Schicht ist bevorzugt aus einem Material gefertigt, das als ein Haftvermittler geeignet ist, d.h. eine Haftung zwischen Außen- und Innenschicht erzeugen kann. Die mittlere Schicht weist bevorzugt das Material linear low density Polyethylen (LLDP) auf. Des Weiteren kann zur Reduktion der Reibung auf die entsprechende Schicht eine Beschichtung eingesetzt werden. Diese Beschichtung kann hydrophob oder hydrophil sein und aus jedem, dem Fachmann für sinnvoll erachtetem Material bestehen.

In einer weiteren Ausgestaltung der Erfindung wird vorgeschlagen, dass das erste Bauteil und/oder das zumindest zweite Bauteil eine Durchführung für eines der Einführelemente aufweist. Dies erlaubt eine kompakte Anordnung, welche das durchgeführte Einführelement stabilisiert und schützt. Ist die Einführvorrichtung ein Katheter, so kann das betreffende Einführelement ein Innenschaft des Katheters sein.

Ferner ist es vorteilhaft, wenn das erste Bauteil am proximalen Ende der Klemmeinheit und das zumindest zweite Bauteil am distalen Ende der Klemmeinheit angeordnet sind. Dadurch können eine Platzierung des Implantats in der Klemmeinheit und eine Freigabe des Implantats einfach erfolgen. Somit ist das zumindest zweite Bauteil bzw. der Stecker in einer Richtung vom proximalen zum distalen Ende der Klemmeinheit bzw. der Einführvorrichtung nach dem ersten Bauteil bzw. der Buchse angeordnet. Hierdurch ein axialer Abstand sollte hier möglichst gering einstellbar sein, um Inhomogenitäten, die eine Stabilität und Funktionalität des Implantats beeinträchtigen könnten, in der Lagerung des Implantats zu vermeiden. Hierbei ist dieser Abstand abhängig von einem Design des verwendeten Implantats und wird vom Fachmann aufgrund seiner Fachkenntnis selbstständig gewählt.

Die Klemmeinheit und die Einführvorrichtung können besonders komfortabel und leicht bedienbar ausgestaltet werden, wenn das erste Bauteil und/oder das zumindest zweite Bauteil axial fixiert auf einem der Einführelemente angeordnet ist. Insbesondere können dadurch das erste Bauteil und/oder das zumindest zweite Bauteil über das Einführelement bedient und/oder bewegt, insbesondere auch für und/oder nach der Freigabe des Implantats zurückgezogen, werden, was eine vorteilhaft kontrollierten Bedienbarkeit der Freigabevorrichtung ermöglicht. Ist die Einführvorrichtung ein Katheter, stellt das Einführelement bevorzugt den Innenschaft dar. Zudem kann das Implantat besonders verliersicher in der Klemmeinheit gehalten werden, wenn ein Innendurchmesser des ersten Bauteils um maximal 0,2 mm breiter als ein Außendurchmesser eines proximalen Endes des Implantats in dessen gestauchten Zustand ist. Hierbei stellt der gestauchte Zustand des Implantats den zusammengedrückten Zustand des Implantats bzw. dessen proximalen Endes unmittelbar vor der Platzierung am ersten Bauteil und insbesondere der Einführung in den Hohlraum des ersten Bauteils dar. Insbesondere ist das Implantat hier schon auf dem Innenschaft und radial um das zweite Bauteil herum platziert und/oder gecrimpt. Grundsätzlich könnte die Durchmesserdifferenz auch geringer sein, hierbei muss jedoch darauf geachtet werden, dass das Implantat stressfrei, insbesondere ohne Schaden zu nehmen, beispielsweise durch irreversible Verformungen, eingeklemmt werden kann.

Des Weiteren kann ein Ende des ersten Bauteils relativ zum anderen Ende in seiner Erstreckung und/oder in seinem Durchmesser verbreitert und/oder aufgeweitet sein. Hierdurch kann das Implantat einfach in das erste Bauteil eingeführt werden. Nach einem Anschlag des eingeführten Endes des Implantats an eine radiale Innenfläche des ersten Bauteils, erfolgt eine Fixierung des Implantats bei weiterem Einführen durch Stauchung einer Erstreckung bzw. eines Durchmessers des Implantats mittels der in axialer Richtung schräg ausgeführten Innenfläche. Das Verbreitern und/oder Aufweiten kann mit jeder, dem Fachmann für anwendbar erachteten Methode erfolgen und/oder ist insbesondere abhängig von einem Material des ersten Baurteils. Bevorzugt ist das distale Ende aufgeweitet, wodurch auch der Hüllbereich bzw. dessen distales Ende aufgeweitet ist. Grundsätzlich könnte das erste Bauteil auch elastisch ausgeführt sein und das Ende könnte im Klemmzustand elastisch verbreitert sein.

In einer alternativen Ausgestaltung weist das erste Bauteil und/oder das zumindest zweite Bauteil eine konische Kontur zur Interaktion mit dem jeweils anderen Bauteil und/oder dem Implantat auf. Dies erlaubt eine kompakte Bauweise der Einführvorrichtung. Bevorzugt ist das erste Bauteil mit einem sich zum proximalen Ende hin verjungenden Hohlraum ausgeführt und/oder das zumindest zweite Bauteil mit einem sich zum proximalen Ende hin verjungenden Anteil, wie beispielsweise einem Kegelstumpf.

Gemäß einem weiteren Aspekt der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine Freigabevorrichtung zum Lösen des medizinisches Implantats, aufweisend eine Klemmeinheit zum Klemmen des Implantats in der Einführvorrichtung mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei die Klemmeinheit ein erstes Bauteil und zumindest ein zweites Bauteil umfasst, die das Implantat im Klemmzustand zwischen sich einklemmen.

Durch die erfindungsgemäße Ausgestaltung kann eine Einführvorrichtung bereitgestellt werden, in der das Implantat sicher gehalten wird bzw. auf dem Innenschaft der Einführvorrichtung sicher befestigt ist. Die Klemmeinheit erlaubt ferner eine kompakte und einfache Konstruktion der Einführvorrichtung. Zudem kann das Implantat einfach auf der Einführvorrichtung bzw. dem Katheter montiert werden. Des Weiteren ist die Freigabe des Implantats zuverlässig und schnell. Ferner kann ein Risiko einer Deformation des Implantats sowie einer daraus resultierende Blockade der Einführvorrichtung, wie sie bei Vorrichtungen des Standes der Technik, die mit Haken und Ösen arbeiten, auftreten, eliminiert werden. Die Einführvorrichtung kann günstigerweise ein Katheter sein. Besonders vorteilhaft kann die Einführvorrichtung zur Montage und Freigabe einer Prothese, einer Herzklappe oder eines Stents eingesetzt werden.

Zudem wird vorgeschlagen, dass die Einführvorrichtung einen Stopper aufweist, der eine Bewegung des Implantats in Richtung eines proximalen Endes der Einführvorrichtung limitiert. Solch eine Bewegung würde unvorteilhaft zu einer Blockade des Implantates führen. Eine bevorzugte Weiterbildung besteht darin, dass die Klemmeinheit den Stopper aufweist, wodurch eine Position der Klemmeinheit während der Bewegung des äußeren Einführelements in Richtung eines proximalen Endes der Einführvorrichtung bzw. der Relativbewegung der beiden Einführelemente klar festgelegt ist. Des Weiteren kann der Stopper mit einem der Bauteile der Klemmeinheit verbunden sein, wodurch ein Verrücken der Klemmeinheit relativ zum Stopper effektiv verhindert werden kann. Hierbei kann jede, dem Fachmann für sinnvoll erachtete Verbindungsart, wie ein Kraft-, ein Form- oder ein Stoffschluss, in Frage kommen. Besonders bevorzugt ist das erste Bauteil der Klemmeinheit mit dem Stopper einstückig ausgeführt ist, wodurch die Anordnung sehr stabil ausgeführt sein kann. Hierbei soll unter "einstückig" verstanden werden, dass der Stopper und das erste Bauteiler von demselben Bauteil gebildet sind und/oder nur unter Funktionsverlust zumindest eines der Bauteile voneinander getrennt werden können.

Gemäß einer vorteilhaften Ausgestaltung kann das Implantat ein selbstexpandierendes Implantat sein, wodurch es die Verbindung mit dem ersten Bauteil durch seine eigene Radialkraft vorteilhaft unterstützen kann. Durch das selbstexpandierende Implantat kann ein zusätzliches Expandiermittel entfallen. Dadurch können vorteilhaft Raum und Montageaufwand für dieses eingespart werden. Hierdurch kann auch die Einführvorrichtung weniger komplex gestaltet werden. Grundsätzlich wäre es jedoch auch möglich ein ballonexpandierbares Implantat zu verwenden. Hierfür müsste die Einführvorrichtung jedoch entsprechend angepasst werden, was der Fachmann aufgrund seiner Fachkenntnis selbstständig löst. Besonders vorteilhaft ist das Implantat befestigungselementlos ausgebildet, wodurch das Implantat gegenüber Implantaten des Standes der Technik verkürzt werden kann. Dies wirkt sich vor allem für den Patienten positiv aus. Folglich kann auch die Einführvorrichtung befestigungselementlos ausgestaltet werden. Hierdurch entfällt bei der Montage des Implantats auf der Einführvorrichtung ein sonst anfälliges Verbinden von Befestigungselementen, wie Haken und Ösen, wodurch auch ein Ausschuss wegen Fehlmontagen Kosten sparend reduziert wird. Dies resultiert vor allem in einer Zeitersparnis bei der Vorbereitung der Einführvorrichtung im Vorbereitungslabor. Des Weiteren entfällt ein im Stand der Technik oft problembehaftetes Lösen der Verbindung zwischen Haken und Ösen während des Implantationsvorgangs. Zudem kann eine hohe Präzision bei der Positionierung des Implantats erreicht werden, statt wie im Stand der Technik Haken und Ösen einzusetzen.

Zudem wird ein Verfahren zum Klemmen eines medizinischen Implantats mittels einer Klemmeinheit einer Freigabevorrichtung in einer Einführvorrichtung, bei welcher das Implantat durch eine Relativbewegung zwischen einem ersten und einem zweiten Einführelement freisetzt wird, vorgeschlagen. Die Klemmeinheit umfasst ein proximales Ende, das im Benutzungszustand von einem distalen Ende der Einführvorrichtung entfernt ist, und einem distalen Ende, das im Benutzungszustand dem distalen Ende der Einführvorrichtung zugewandt ist. Das Verfahren weist zumindest die folgenden Schritte auf: Platzieren des Implantats an einem Einführelement und zumindest eines Teils des Implantats über zumindest einem zweiten Bauteil der Klemmeinheit, so dass zumindest ein proximales Ende des Implantats proximal vom zweiten Bauteil angeordnet ist; Biegen zumindest eines proximalen Endes des Implantats radial in Richtung einer Innenachse des Implantats; Einführen zumindest des proximalen gebogenen Endes des Implantats in ein erstes Bauteil der Klemmeinheit, so dass zumindest ein Bereich des Implantats an zumindest einem Bereich einer Außenfläche des zweiten Bauteils radial anliegt und Platzierung der Klemmeinheit mit dem Implantat in zumindest einem Einführelement.

Durch die erfindungsgemäße Ausgestaltung kann ein Verfahren realisiert werden, mittels dem ein Implantat bedienerfreundlich, präzise, sicher und schnell in der Einführvorrichtung platziert und befestigt werden kann. Auch die Freigabe eines so montierten Implantats erfolgt bedienerfreundlich, präzise, sicher und schnell. Das Platzieren des Implantats auf dem Einführelement erfolgt bevorzugt mittels eines Befestigens und insbesondere mittels eines Crimpprozesses. Ferner kann vor einem Platzieren des Implantats dessen proximales Ende radial nach außen gebogen werden, um die radiale Erstreckung des zweiten Bauteils zu überwinden. Nach dem Platzieren/Befestigen/Crimpen des Implantats bzw. dem Platzieren des Teils des Implantats und dem Überwinden des zweiten Bauteils kann dann das Biegen zumindest des proximalen Endes des Implantats radial in Richtung der Innenachse des Implantats erfolgen. Es wäre auch möglich, dass das zweite Bauteil mittels des radialen Anliegens des zweiten Bauteils über zumindest dem Bereich der Außenfläche an dem Implantat verliersicher in der Einführvorrichtung gehalten wird. In diesem Zusammenhang soll unter "einem Bereich des Implantats" zumindest ein Bereich des Implantats verstanden werden, der unmittelbar, in Richtung des distalen Endes, am proximalen Ende des Implantats anliegt. Grundsätzlich könnte der Bereich auch das proximale Ende und/oder eine Bereich in einer Mitte des Implantats sein. Bevorzugt liegt der Bereich des Implantats an der ganzen Außenfläche des zumindest zweiten Bauteils radial an. Unter der Wendung "Aufschieben zumindest eines Teils des Implantats über zumindest ein zweites Bauteil der Klemmeinheit" soll hier verstanden werden, dass nicht das komplette Implantat über das zweite Bauteil geschoben wird, sondern nur einen Teil und insbesondere zumindest das proximale Ende. Das zweite Bauteil ist somit radial im Implantat angeordnet.

Die Erfindung ist nachfolgend beispielhaft, anhand von in Zeichnungen dargestellten Ausführungsbeispielen, näher erläutert. Es zeigen in schematischer Darstellung:
- Fig. 1: einen Längsschnitt durch ein günstiges Ausführungsbeispiel einer erfindungsgemäßen Freigabevorrichtung mit einem Implantat platziert in einer Einführvorrichtung;
- Fig. 2: eine schematische Darstellung von zwei Bauteilen der Freigabevorrichtung und dem Implantat aus Fig. 1;
- Fig. 3: die Freigabevorrichtung und das Implantat der Fig. 1 in einem Zustand vor einer Platzierung des Implantats in der Einführvorrichtung;
- Fig. 4: die Freigabevorrichtung und das Implantat der Fig. 1 nach einem Platzieren und Biegen des Implantats an der Einführvorrichtung;
- Fig. 5: einen Schnitt durch ein distales Ende der Einführvorrichtung der Fig. 1 mit darin angeordneter Freigabevorrichtung mit platziertem Implantat und
- Fig. 6: eine alternative Freigabevorrichtung und ein Implantat in einem Zustand vor einer Platzierung des Implantats in einer Einführvorrichtung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

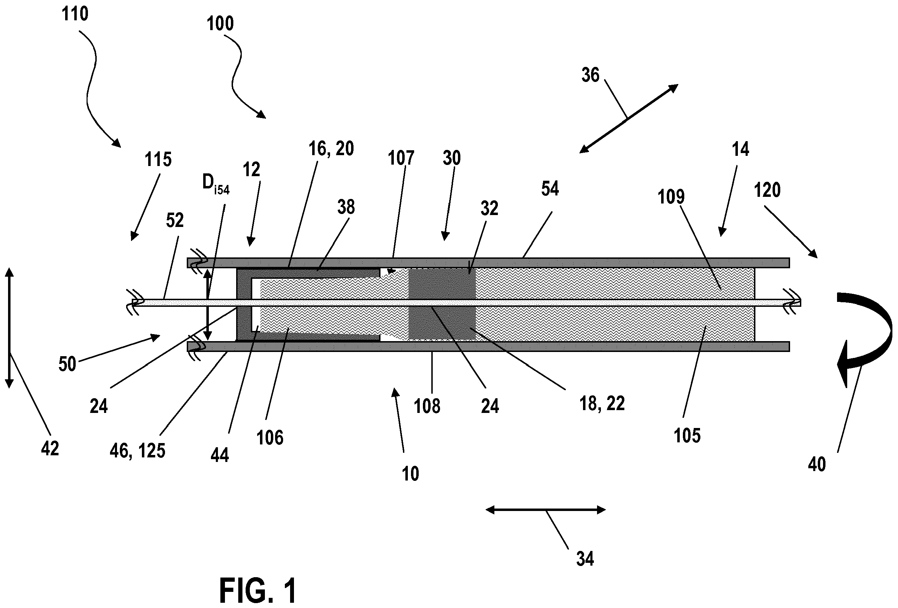

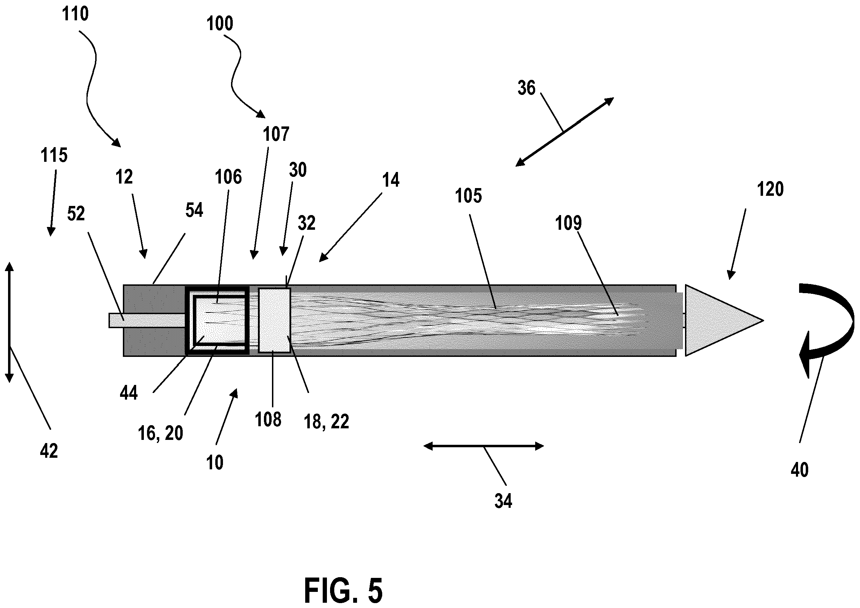

Fig. 1 zeigt einen Längsschnitt durch ein günstiges Ausführungsbeispiel einer erfindungsgemäßen Freigabevorrichtung 100 einer nur teilweise dargestellten Einführvorrichtung 110. Die Einführvorrichtung 110 ist beispielsweise ein Katheter mit einem Schaftbereich 50 mit zwei koaxial angeordneten Einführelementen 52, 54, z.B. einem Innenschaft (Einführelement 52) und einem diesen umgebenden Außenschaft (Einführelement 54), welcher wiederum von einer nicht gezeigten Außenhülle umgeben sein kann. Die Einführvorrichtung 110 ist im Anwenderbetrieb, also während einer Befestigung eines Implantats 105 an der Freigabevorrichtung 100 oder während der Implantation mit ihrem proximalen Ende 115 einem Anwender zugewandt. Das Implantat 105 ist am distalen Ende 120 des Schaftbereichs 50 zwischen Innenschaft und Außenschaft platziert und soll am Implantationsort im tierischen oder menschlichen Körper freigegeben werden (vgl. Fig. 5).

Die Freigabevorrichtung 100 dient zum Lösen des medizinischen Implantats 105 von der Einführvorrichtung 110. Das Implantat 105 ist an einem vom Anwender abgewandten Ende 120 des Schaftbereichs 50 beispielsweise in der Nähe einer Katheterspitze angeordnet (siehe Fig. 5). Das Implantat 105 ist beispielsweise um das innere Einführelement 52 gelegt und wird durch eine Relativbewegung zwischen dem ersten und dem zweiten Einführelement 52, 54 freigesetzt.

Die Freigabevorrichtung 100 umfassend eine Klemmeinheit 10 zum Klemmen des Implantats 105 in der Einführvorrichtung 110. Zudem weist die Klemmeinheit 10 ein proximales Ende 12, das im Benutzungszustand von dem distalen Ende 120 der Einführvorrichtung 110 entfernt ist, und ein distales Ende 14, das im Benutzungszustand dem distalen Ende 120 der Einführvorrichtung 110 zugewandt ist, auf. Des Weiteren umfasst die Klemmeinheit 10 ein erstes Bauteil 16 und ein zweites Bauteil 18, die das Implantat 105 im Klemmzustand in der Klemmeinheit 10 in einer longitudinalen bzw. axialen Richtung 34 und einer transversalen Richtung 36 zwischen sich einklemmen. Im Klemmzustand hält ein Formschuss zwischen dem ersten Bauteil 16 und dem Implantat 105 und eine Reibung zwischen dem zweiten Bauteil 18 und dem Implantat 105 dieses in Position.

Für den Formschluss mit dem Implantat 105 ist das erste Bauteil 16 als eine Buchse 20 ausgebildet. Hierbei stellt einer in Richtung des distalen Endes 14 der Klemmeinheit 10 angeordneter zylinderförmiger Anteil der Buchse 20 einen Hüllbereich 38 dar, der im Klemmzustand ein proximales Ende 106 des Implantats 105 in einer Umfangsrichtung 40 umgibt bzw. diese in transversaler Richtung 36 und radialer Richtung 42 einklemmt. Der Hüllbereich 38 formt einen Hohlraum 44 in dem das proximale Ende 106 des Implantats 105 aufgenommen und angeordnet werden kann bzw. im Klemmzustand angeordnet ist. An einem in Richtung des proximalen Endes 12 der Klemmeinheit 10 angeordneten Bereich des ersten Bauteils 16 ist eine sich in radialer Richtung 42 erstreckende Wandung 46 angeformt.

Das zweite Bauteil 18 ist von einem zylinderförmigen Bauteil gebildet und stellt einen Stecker 22 dar, der im Implantat 105 angeordnet werden kann bzw. im Klemmzustand im Implantat 105 angeordnet ist. Hierbei liegt ein Bereich 108 des Implantats 105 an einem Bereich 30 einer Außenfläche 32, hier der gesamten radialen Außenfläche 32, des zweiten Bauteils 18 bzw. dem Stecker 22 radial an. Durch diese Anordnung wird der Bereich 108 des Implantats 105 in transversaler Richtung 36 und radialer Richtung 42 an das Einführelement 54 bzw. den Außenschaft gedrückt und zwischen diesem und dem Stecker 22 eingeklemmt. Das erste Bauteil 16 ist am proximalen Ende 12 und das zweite Bauteil 18 am distalen Ende 14 der Klemmeinheit 10 angeordnet. Somit ist in einer axialen 34 Richtung vom proximalen 12 zum distalen Ende 14 der Klemmeinheit 10 bzw. der Einführvorrichtung 110 das zweite Bauteil 18 bzw. der Stecker 22 nach dem ersten Bauteil 16 bzw. der Buchse 20 angeordnet. Beide Bauteile 16, 18 sind auf dem Einführelement 52 bzw. dem Innenschaft in axialer Richtung 34 fixiert angeordnet, wodurch diese mit dem Einführelement 52 bewegt werden können. Hierfür weisen das erste Bauteil 16 und das zweite Bauteil 18 je eine Durchführung 24 für das Einführelement 52 bzw. den Innenschaft auf.

Für eine erforderliche Haftung zwischen dem Implantat 105 und dem zweiten Bauteil 18 bzw. dem Stecker 22, insbesondere um das Implantat 105 im Klemmzustand in der Einführvorrichtung 110 in Position zu halten, ist dieses/dieser aus einem Monolayer-Design Polymer (z.B. PEBAX der Firma Arkema) mit hoher Haftreibung gefertigt. Das erste Bauteil 16 ist hingegen aus einem Material gefertigt, das härter ist als das Material des zweiten Bauteils 18. Hierdurch kann die Buchse 20 leicht im Einführelement 54 bewegt werden. Das Material des ersten Bauteils 16 ist beispielsweise ein harter Kunststoff, wie Polycarbonat. Alternativ wäre es auch möglich, das erste Bauteil 16 mit einem so genannten Multilayer-Co-extrudiertem Design mit einer Außenschicht und einer Innenschicht zu fertigen (nicht gezeigt). Hierbei wären die Außenschicht aus einem Material mit geringer Reibung (z.B. PC) und die Innenschicht aus einem Material mit höherer Reibung (z.B. PEBAX) gefertigt. Durch die hohe Reibung des Materials der Innenschicht kann der Formschluss zwischen dem ersten Bauteil und dem proximalen Ende des Implantats unterstütz werden.

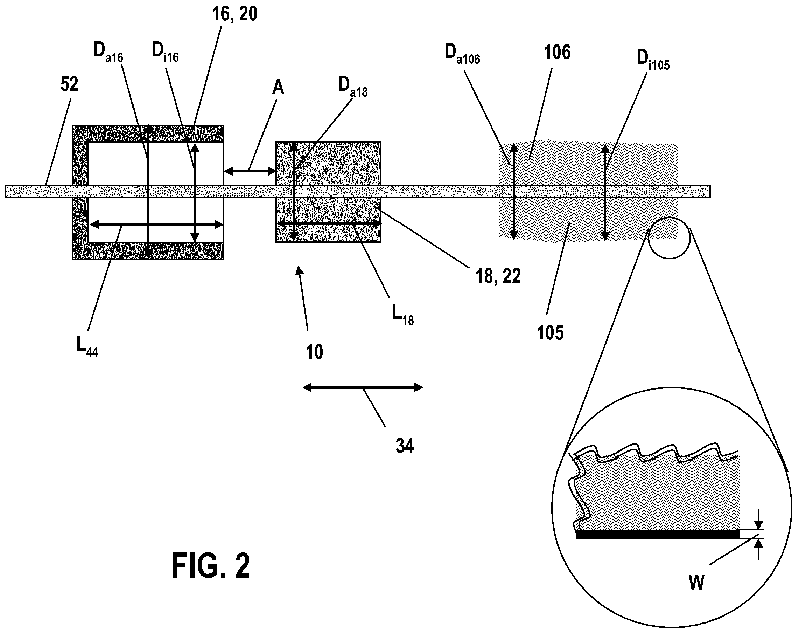

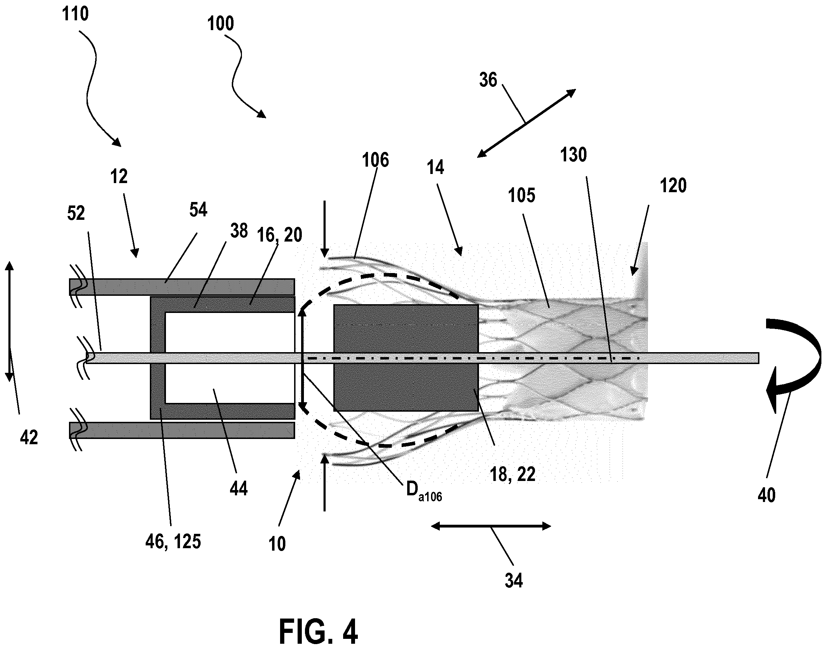

In der Fig. 2 sind die zwei Bauteile 16, 18 der Freigabevorrichtung 100 und das Implantat 105 schematisch gezeigt. Das erste und das zweite Bauteil 16, 18 weisen jeweils einen im Wesentlichen runden Querschnitt auf (nicht im Detail gezeigt). Ein Innendurchmesser Dᵢ₁₆ des ersten Bauteils 16 bzw. dessen Hohlraums 44 ist an eine Kontur oder einen Außendurchmesser des verwendeten Implantats 105 angepasst und ist somit von dessen Dimensionen und Eigenschaften abhängig. Grundsätzlich sollte er so klein wie möglich sein, z.B. zwischen 0,1 mm bis maximal 0,2 mm breiter als ein Außendurchmesser Dₐ₁₀₆ des proximalen Endes 106 des Implantats 105 im gestauchten Zustand des gecrimpten Implantats 105 vor dessen Einführung in den Hohlraum 44 des ersten Bauteils 16 (vgl. Fig. 4). Eine Länge L₄₄ des Hohlraums 44 ist ebenso abhängig vom verwendeten Implantat 105 und kann beispielsweise 5 mm bis 10 mm betragen. Des Weiteren sind ein Außendurchmesser Dₐ₁₆ des ersten Bauteils 16 auf Maße der Einführvorrichtung 110, wie beispielsweise einem Innendurchmesser Dᵢ₅₄ des Einführelements 54 bzw. des Außenschafts, abgestimmt (vgl. Fig. 1). Hierbei kann beispielsweise ein Außendurchmesser Dₐ₁₆ des ersten Bauteils 16 der Innendurchmesser Dᵢ₅₄ des Außenschafts subtrahiert um 0,2 mm sein, wodurch das erste Bauteil 16 im montierten Zustand in der Einführvorrichtung 110 mit einem Radialspalt von 0,1 mm angeordnet ist.

Ein Außendurchmesser Dₐ₁₈ des zweiten Bauteils 18 ist der Innendurchmesser Dᵢ₅₄ des Außenschafts verringert um zweimal eine Wandstärke W des verwendeten Implantats 105 und ist somit abhängig von Dimensionen, wie einem Innendurchmesser Dᵢ₁₀₅, und Eigenschaften des Implantats 105. Eine Länge L₁₈ des zweiten Bauteils 18 bzw. des Steckers 22 kann beispielsweise 5 mm betragen. Ein axialer Abstand A zwischen dem ersten Bauteil 16 und dem zweiten Bauteil 18 ist abhängig von den Dimensionen und Eigenschaften des Implantats 105 und soll so kurz wie möglich sein. Er kann beispielsweise 1 mm bis 5 mm betragen.

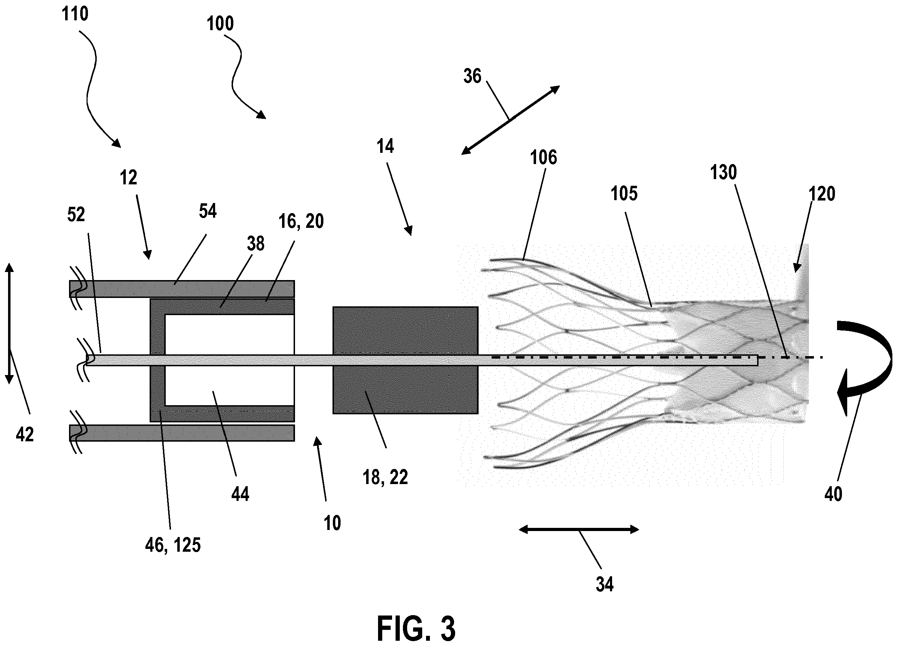

In Fig. 3 ist die Einführvorrichtung 110 vor der Platzierung des Implantats 105 in der Klemmeinheit 10 gezeigt. Hierfür wurde das äußere Einführelement 54 in Richtung des proximalen Endes 115 der Einführvorrichtung 110 verschoben, bis das erste Bauteil 16 an einem Rand des äußeren Einführelements 54, der zum distalen Ende 120 der Einführvorrichtung 110 weist, frei liegt. Das erste und das zweite Bauteil 16, 18 können entweder in einer Einführvorrichtung 110 vormontiert sein, beispielsweise von einem Hersteller, oder sie können auch erst kurz vor einer Montage des Implantats 105 im Katheterlabor montiert werden. Zu einer Limitierung der Bewegung des Implantats 105 in Richtung des proximalen Endes 115 der Einführvorrichtung 110 bei der Montage weist die Einführvorrichtung 110 einen Stopper 125 auf. Dieser Stopper 125 wird von der Wandung 46 gebildet und ist somit einstückig mit dem ersten Bauteil 16 der Klemmeinheit 10 ausgeführt. Das Implantat 105, beispielsweise ein Stent oder ein künstliches Herzklappenimplantat, ist selbstexpandierend und befestigungselementlos, also beispielsweise ohne Haken oder Ösen, ausgebildet.

Im Folgenden ist ein Verfahren zum Klemmen des Implantats 105 mittels der Klemmeinheit 10 anhand der Fig. 4 und 5 beschrieben. In einem ersten Schritt wird das Implantat 105 platziert. Hierbei wird das gesamte Implantat 105 über das innere Einführelement 52 und ein Teil 107 des Implantats 105 über das zweite Bauteil 18 der Klemmeinheit 10 geschoben, so dass zumindest das proximale Ende 106 des Implantats 105 proximal vom zweiten Bauteil 18, axial zwischen dem ersten und dem zweiten Bauteil 16, 18, angeordnet ist (siehe Fig. 4). Ist das Implantat 105 platziert, wird es auf dem inneren Einführelement 52 mittels Crimpen befestigt. Wie in Fig. 4 ebenso dargestellt ist, wird in einem nachfolgenden, zweiten Schritt das proximale Ende 106 des Implantats 105 radial in Richtung einer Innenachse 130 des Implantats 105 gebogen (siehe Pfeile), wodurch das proximale Ende 106 des Implantats 105 die schematisch gezeigte, gestrichelte Position einnimmt. In einem dritten Schritt wird das so gebogene Implantat 105 bzw. dessen proximal gebogenes Endes 106 aus Richtung des distalen Endes 14 in den Hohlraum 44 des ersten Bauteils 16 der Klemmeinheit 10 eingeführt. Das Implantat 105 wird dabei so platziert, dass der Bereich 108 des Implantats 105 an dem Bereich 30 der Außenfläche 32 des zweiten Bauteils 18, insbesondere an der gesamten radialen Außenfläche 32, radial anliegt (siehe Fig. 5). Der Teil 107 stellt das proximale Ende 106 des Implantats 105 und den distal daran anschließenden Teil 108 dar. Das Implantat 105 wird nun durch den Formschluss zwischen dem ersten Bauteil 16 und dem Implantat 105 an dem inneren Einführelement 52 in Position gehalten. In einem letzten Schritt wird das äußere Einführelement 54 über die Klemmeinheit 10 geschoben, wodurch die Klemmeinheit 10 mit dem Implantat 105 in dem Einführelement 54 platziert wird (siehe Fig. 5). Das Implantat 105 wird nun einerseits durch den Formschluss zwischen dem ersten Bauteil 16 und dem Implantat 105 und durch die Wechselwirkung des Implantats 105 mit einer Innenfläche des äußeren Einführelements 54 in Position gehalten. Diese sichere Position wird bei der Bewegung der Klemmeinheit 10 und das Implantats 105 in dem äußeren Einführelement 54 durch die Haftreibung zwischen dem zweiten Bauteil 18 und dem Implantat 105 weiter unterstützt, wodurch ein Entgleiten des Implantats 105 sicher verhindert wird.

Zu einer Implantation des Implantats 105 im Körper, wird die so vorbereitete Einführvorrichtung 110 in den Körper eingeführt. Durch die Bewegung des äußeren Einführelements 54 in Richtung des proximalen Endes 115 der Einführvorrichtung 110 wird bei Freilegung eines distalen Endes 109 des Implantats 105 zuerst dieses wegen seiner Fähigkeit der Selbstexpansion geöffnet und positioniert. Wird der Außenschaft und der Innenschaft mit dem daran befestigten ersten und zweiten Bauteil 16, 18 zurückgezogen, wird auch das proximale Ende 106 des Implantats 105 freigelegt. Hierdurch wird auch das proximale Ende 106 des Implantats 105 aufgrund seiner Radialkraft geöffnet. Im Anschluss wird die Freigabevorrichtung 100 bzw. die Klemmeinheit 10 mit dem Innenschaft in den Außenschaft zurückgezogen und die Einführvorrichtung 110 aus dem Körper entfernt. Das Implantat 105 verbleibt vollständig positioniert im Körper (nicht gezeigt).

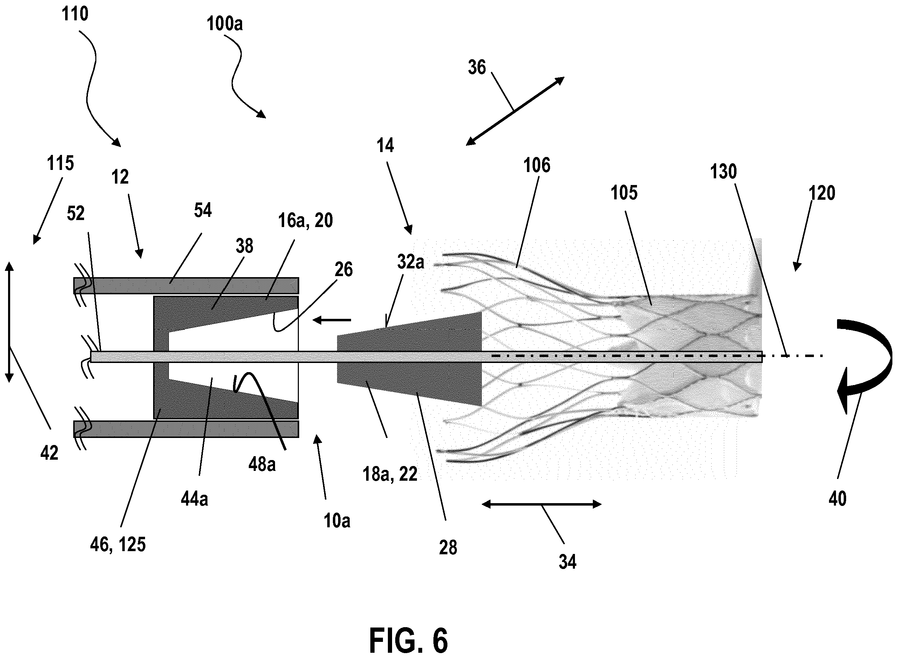

In Fig. 6 ist ein alternatives Ausführungsbeispiel der Freigabevorrichtung 100a dargestellt. Im Wesentlichen sind gleich bleibende Bauteile, Merkmale und Funktionen grundsätzlich mit den gleichen Bezugszeichen beziffert. Zur Unterscheidung des Ausführungsbeispiels der Fig. 6 zu dem in den Fig. 1 bis 5 sind jedoch den Bezugszeichen der in dem Ausführungsbeispiel der Fig. 6 abweichenden ausgeführten Bauteile der Buchstabe a hinzugefügt. Die nachfolgende Beschreibung beschränkt sich im Wesentlichen auf die Unterschiede zu dem Ausführungsbeispiel in den Fig. 1 bis 5, wobei bezüglich gleich bleibender Bauteile, Merkmale und Funktionen auf die Beschreibung des Ausführungsbeispiels in den Fig. 1 bis 5 verwiesen werden kann.

Die Freigabevorrichtung 100a der Fig. 6 unterscheidet sich von der Freigabevorrichtung 100 der Fig. 1 bis 5 dadurch, dass ein erstes Bauteil 16a und ein zweites Bauteil 18a einer Klemmeinheit 10a je eine konische Kontur 26, 28 zur Interaktion mit dem jeweils anderen Bauteil 16a, 18a und einem Implantat 105 aufweist. Die konische Kontur 26 des ersten Bauteils 16a wird von zwei in Richtung eines proximalen Endes 12 der Klemmeinheit 10a aufeinander zulaufenden radialen Innenflächen 48a eines Hohlraums 44a des ersten Bauteils 16a gebildet. Das zweite Bauteil 18a weist als konische Kontur 28 zwei in Richtung des proximalen Endes 12 aufeinander zulaufenden Außenflächen 32a auf, die zu den zwei Innenflächen 48a des ersten Bauteils 16a korrespondierend ausgeführt sind. Bei dieser Ausgestaltung ist das zweite Bauteil18a in axialer Richtung 34 verschieblich auf einem inneren Einführelement 52 einer Einführvorrichtung 110 angeordnet und kann in den Hohlraum 44a des ersten Bauteils 16a aus Richtung eines distalen Endes 14 der Klemmeinheit 10a eingeführt werden. Dadurch wird das Implantat 105 bzw. ein proximales Ende 106 des Implantats 105 zwischen den Innenflächen 48a des ersten Bauteils 16a und den Außenflächen 32a des zweiten Bauteils 18a eingeklemmt und das Implantat 105 wird zusätzlich durch das zweite Bauteil 18a im Hohlraum 44a gehalten. Durch die fehlende Verbindung zwischen dem zweiten Bauteil 18a und dem inneren Einführelement 52 muss das Einführelement 52 ein nicht näher gezeigtes Mitnahmeelement aufweisen, um das zweite Bauteil 18a nach einer Freigabe des Implantats 105 im Körper aus diesem entfernen zu können. Dieses Mitnahmeelement könnte, im Falle der Ausführung der Einführeinheit als Katheter, von einer Katheterspitze gebildet sein.

## Patentansprüche

1. Freigabevorrichtung (100, 100a) zum Lösen eines selbstexpandierenden medizinischen Implantats (105) von einer Einführvorrichtung (110), bei welcher das selbstexpandierende Implantat (105) durch eine Relativbewegung zwischen einem Innenschaft (52) und einem Außenschaft (54) und das anschließende Zurückziehen der Einführvorrichtung (110) freisetzbar ist, wobei die Relativbewegung zwischen dem Innenschaft (52) und dem Außenschaft (54) eine Bewegung des Außenschafts (54) in Richtung des proximalen Endes (115) der Einführvorrichtung (110) umfasst, umfassend eine Klemmeinheit (10, 10a) zum Klemmen des selbstexpandierenden Implantats (105) in der Einführvorrichtung (110), mit einem proximalen Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, wobei die Klemmeinheit (10, 10a) ein erstes Bauteil (16, 16a) und zumindest ein zweites Bauteil (18, 18a) umfasst,
wobei das erste Bauteil (16, 16a) am proximalen Ende (12) der Klemmeinheit (10, 10a) und das zweite Bauteil (18, 18a) am distalen Ende (14) der Klemmeinheit (10, 10a) angeordnet sind,
wobei das erste Bauteil (16, 16a) und das zweite Bauteil (18, 18a) axial fixiert auf dem Innenschaft (52) angeordnet sind,
wobei das selbstexpandierende Implantat (105) im Klemmzustand zwischen dem Außenschaft (54) und dem zweiten Bauteil (18, 18a) eingeklemmt ist, wobei das erste Bauteil (16, 16a) einen Hüllenbereich (38) aufweist,
wobei der Hüllenbereich (38) im Klemmzustand ein proximales Ende (106) des selbstexpandierenden Implantats (105) in einer Umfangsrichtung (40) umgibt und dadurch einen Formschluss zwischen dem ersten Bauteil (16, 16a) und dem selbstexpandierenden Implantat (105) erzeugen kann, der das selbstexpandierende Implantat (105) in Position hält, **dadurch gekennzeichnet, dass** das erste Bauteil (16, 16a) ein Material aufweist, das härter ist als das Material des zumindest zweiten Bauteils (18, 18a), wodurch das erste Bauteil (16, 16a) Radialkräften des zweiten Bauteils und/oder des Implantats stand halten kann.

2. Freigabevorrichtung nach Anspruch 1, wobei im Klemmzustand eine Reibung zwischen dem zumindest zweiten Bauteil (18, 18a) und dem Implantat (105) dieses in Position hält.

3. Freigabevorrichtung nach Anspruch 1 oder 2, wobei das erste Bauteil (16, 16a) von einer Buchse (20) und/oder das zumindest zweite Bauteil (18, 18a) von einem Stecker (22) gebildet ist.

4. Freigabevorrichtung nach einem der vorhergehenden Ansprüche, wobei das zumindest zweite Bauteil (18, 18a) ein Material mit hoher Haftreibung aufweist, um das selbstexpandierende Implantat (105) in dem Klemmzustand in Position zu halten.

5. Freigabevorrichtung nach Anspruch 4, wobei das Material ein Polymer und insbesondere ein Material ausgewählt aus der Gruppe bestehend aus Polyamid, Polyester, Polyether-Block-Amid, Silikon, Polyurethan ist.

6. Freigabevorrichtung nach einem der vorhergehenden Ansprüche, wobei das Material des ersten Bauteils (16, 16a) ein Material ausgewählt aus der Gruppe bestehend aus einem Kunststoff, insbesondere Polycarbonat, Polyetheretherketon (PEEK), Perfluor-Ethylen-Propylen, high density Polyethylen, Polytetrafluorethylen, Polyamid, ein Metall, eine Keramik, ein Hart-Gummi oder ein Glas ist.

7. Freigabevorrichtung nach Anspruch 6, wobei das Material des ersten Bauteils (16, 16a) ein Metall ist, bevorzugt ein rostfreier Stahl, insbesondere mit einer strukturierten Oberfläche.

8. Freigabevorrichtung nach einem der vorhergehenden Ansprüche, wobei das erste Bauteil (16, 16a) und/oder das zumindest zweite Bauteil (18, 18a) eine Durchführung (24) für den Innenschaft (52) aufweist.

9. Freigabevorrichtung nach einem der vorhergehenden Ansprüche, wobei das erste Bauteil (16a) und/oder das zumindest zweite Bauteil (18a) eine konische Kontur (28, 30) zur Interaktion mit dem selbstexpandierenden Implantat (105) aufweist.

10. System umfassend eine Freigabevorrichtung (100, 100a) nach einem der vorhergehenden Ansprüchen und ein in der Freigabevorrichtung (100, 100a) eingeklemmtes selbstexpandierendes Implantat (105).

11. System nach Anspruch 10, wobei ein Innendurchmesser (Dᵢ₁₆) des ersten Bauteils (16, 16a) um maximal 0,2 mm breiter ist als ein Außendurchmesser (Dₐ₁₀₆) eines proximalen Endes (106) des Implantats (105) in dessen gestauchten Zustand.

12. System nach Anspruch 10 oder 11, wobei das erste Bauteil (16, 16a) zumindest zwei Schichten aufweist, die in radialer Richtung übereinander angeordnet sind, wobei die radial äußere Schicht ein Material mit geringer Reibung zum Außenschaft (54) aufweist und die radial innere Schicht ein Material mit hoher Reibung zum selbstexpandierenden Implantat (105) aufweist.

13. Einführvorrichtung (110) zum Einführen eines medizinischen selbstexpandierenden Implantats (105) umfassend eine Freigabevorrichtung (100, 100a) zum Lösen des medizinisches selbstexpandierenden Implantats (105), nach einem der Ansprüche 1 bis 9.

14. Einführvorrichtung nach Anspruch 13, wobei ein Stopper (125) vorgesehen ist, der eine Bewegung des selbstexpandierenden Implantats (105) in Richtung eines proximalen Endes (115) der Einführvorrichtung (110) limitiert, wobei die Klemmeinheit (10, 10a) den Stopper (125) aufweist und/oder das erste Bauteil (16, 16a) der Klemmeinheit (10, 10a) mit dem Stopper (125) einstückig ausgeführt ist.

15. Einführvorrichtung nach Anspruch 13 oder 14, wobei das selbstexpandierende Implantat (105) befestigungselementlos ausgebildet ist.

16. Verfahren zum Klemmen eines selbstexpandierenden medizinischen Implantats (105) mittels einer Klemmeinheit (10, 10a) einer Freigabevorrichtung (100, 100a), nach einem der Ansprüche 1 bis 9, in einer Einführvorrichtung (110) nach Anspruch 13, bei welcher das selbstexpandierende Implantat (105) durch eine Relativbewegung zwischen einem Innenschaft (52) und einem Außenschaft (54) und das anschließende Zurückziehen der Einführvorrichtung (110) freigesetzt wird, wobei die Klemmeinheit (10, 10a) ein proximales Ende (12), das im Benutzungszustand von einem distalen Ende (120) der Einführvorrichtung (110) entfernt ist, und ein distales Ende (14), das im Benutzungszustand dem distalen Ende (120) der Einführvorrichtung (110) zugewandt ist, umfasst, aufweisend zumindest die folgenden Schritte:
- Platzieren des selbstexpandierenden Implantats (105) an einem Innenschaft (52) und zumindest eines Teils (107) des selbstexpandierenden Implantats (105) über zumindest einem zweiten Bauteil (18, 18a) der Klemmeinheit (10), so dass zumindest ein proximales Ende (106) des selbstexpandierenden Implantats (105) proximal vom zweiten Bauteil (18, 18a) angeordnet ist,
- Biegen zumindest eines proximalen Endes (106) des selbstexpandierenden Implantats (105) radial in Richtung einer Innenachse (130) des selbstexpandierenden Implantats (105);
- Einführen zumindest des proximalen gebogenen Endes (106) des selbstexpandierenden Implantats (105) in einen Hüllenbereich (38) des ersten Bauteils (16, 16a) der Klemmeinheit (10), so dass zumindest ein Bereich (108) des selbstexpandierenden Implantats (105) an zumindest einem Bereich (30) einer Außenfläche (32, 32a) des zweiten Bauteils (18, 18a) radial anliegt;
- Platzierung der Klemmeinheit (10) mit dem selbstexpandierenden Implantat (105) in zumindest einen Außenschaft (54).

## Claims

1. A release device (100, 100a) for releasing a medical self-expanding implant (105) from an insertion device (110), in which the self-expanding implant (105) can be released by way of a relative movement between an inner shaft (52) and an outer shaft (54) and the subsequent retraction of the insertion device (110), the relative movement between the inner shaft (52) and the outer shaft (54) comprising a movement of the outer shaft (54) in the direction of the proximal end (115) of the insertion device (110), the release device comprising a clamping unit (10, 10a) for clamping the self-expanding implant (105) in the insertion device (110), having a proximal end (12), which is distant from a distal end (120) of the insertion device (110) in the state of use, and having a distal end (14), which faces the distal end (120) of the insertion device (110) in the state of use, the clamping unit (10, 10a) comprising a first component (16, 16a) and at least one second component (18, 18a), the first component (16, 16a) being arranged at the proximal end (12) of the clamping unit (10, 10a) and the second component (18, 18a) being arranged at the distal end (14) of the clamping unit (10, 10a),
the first component (16, 16a) and the second component (18, 18a) being arranged on the inner shaft (52) in an axially fixed manner,
the self-expanding implant (105) being clamped between the outer shaft (54) and the second component (18, 18a) in the clamped state,
the first component (16, 16a) having a sleeve region (38),
the sleeve region (38) in the clamped state surrounding a proximal end (106) of the self-expanding implant (105) in a circumferential direction (40) and thus being able to produce a form-fit connection between the first component (16, 16a) and the self-expanding implant (105), which holds the self-expanding implant (105) in position, **characterised in that** the first component (16, 16a) comprises a material that is harder than the material of the at least second component (18, 18a), whereby the first component (16, 16a) can withstand radial forces of the second component and/or the implant.

2. The release device according to claim 1, wherein, in the clamped state, a friction between the at least second component (18, 18a) and the implant (105) holds the latter in position.

3. The release device according to claim 1 or 2, wherein the first component (16, 16a) is formed by a socket (20) and/or the at least second component (18, 18a) is formed by a plug connector (22).

4. The release device according to one of the preceding claims, wherein the at least second component (18, 18a) comprises a material of high static friction to hold the self-expanding implant (105) in position in the clamped state.

5. The release device according to claim 4, wherein the material is a polymer and in particular a material selected from the group consisting of polyamide, polyester, polyether block amide, silicone, polyurethane.

6. The release device according to one of the preceding claims, wherein the material of the first component (16, 16a) is a material selected from the group consisting of a plastic, in particular polycarbonate, polyether ether ketone (PEEK), perfluoroethylene propylene, high-density polyethylene, polytetrafluoroethylene, polyamide, a metal, a ceramic, a hard rubber or a glass.

7. The release device according to claim 6, wherein the material of the first component (16, 16a) is a metal, preferably a stainless steel, in particular with a structured surface.

8. The release device according to one of the preceding claims, wherein the first component (16, 16a) and/or the at least second component (18, 18a) has a passage (24) for the inner shaft (52).

9. The release device according to one of the preceding claims, wherein the first component (16a) and/or the at least second component (18a) has a conical contour (28, 30) for interaction with the self-expanding implant (105).

10. A system comprising a release device (100, 100a) according to one of the preceding claims and a self-expanding implant (105) clamped in the release device (100, 100a).

11. The system according to claim 10, wherein an inner diameter (Dᵢ₁₆) of the first component (16, 16a) is wider, by a maximum of 0.2 mm, than an outer diameter (Dₐ₁₀₆) of a proximal end (106) of the implant (105) in the compressed state thereof.

12. The system according to claim 10 or 11, wherein the first component (16, 16a) has at least two layers, which are arranged one on top of the other in the radial direction, the radially outer layer comprising a material of low friction relative to the outer shaft (54), and the radially inner layer comprising a material of high friction relative to the self-expanding implant (105).

13. An insertion device (110) for inserting a medical self-expanding implant (105) comprising a release device (100, 100a) for releasing the medical self-expanding implant (105) according to one of claims 1 to 9.

14. The insertion device according to claim 13, wherein a stopper (125) is provided, which limits a movement of the self-expanding implant (105) in the direction of a proximal end (115) of the insertion device (110), the clamping unit (10, 10a) comprising the stopper (125) and/or the first component (16, 16a) of the clamping unit (10, 10a) being designed in one piece with the stopper (125).

15. The insertion device according to claim 13 or 14, wherein the self-expanding implant (105) is designed without any fastening elements.

16. A method for clamping a medical self-expanding implant (105) by means of a clamping unit (10, 10a) of a release device (100, 100a) according to one of claims 1 to 9 in an insertion device (110) according to claim 13, in which the self-expanding implant (105) is released by a relative movement between an inner shaft (52) and an outer shaft (54) and the subsequent retraction of the insertion device (110), the clamping unit (10, 10a) having a proximal end (12), which is distant from a distal end (120) of the insertion device (110) in the state of use, and having a distal end (14), which in the state of use faces the distal end (120) of the insertion device (110), the method having at least the following steps:
- placing the self-expanding implant (105) on an inner shaft (52) and placing at least a part (107) of the self-expanding implant (105) over at least one second component (18, 18a) of the clamping unit (10), so that at least a proximal end (106) of the self-expanding implant (105) is disposed proximally of the second component (18, 18a);
- bending at least a proximal end (106) of the self-expanding implant (105) radially in the direction of an inner axis (130) of the self-expanding implant (105);
- inserting at least the proximal bent end (106) of the self-expanding implant (105) into a sleeve region (38) of the first component (16, 16a) of the clamping unit (10), so that at least a region (108) of the self-expanding implant (105) rests radially against at least a region (30) of an outer surface (32, 32a) of the second component (18, 18a), and;
- placing the clamping unit (10) comprising the self-expanding implant (105) in at least one outer shaft (54).

## Revendications

1. Dispositif de libération (100, 100a) permettant de libérer un implant auto-expansible (105) médical d'un dispositif d'insertion (110), chez lequel l'implant auto-expansible (105) peut être libéré par un déplacement relatif entre une tige intérieure (52) et une tige extérieure (54) et le retrait consécutif du dispositif d'insertion (110), où le déplacement relatif entre la tige intérieure (52) et la tige extérieure (54) comprend un déplacement de la tige extérieure (54) en direction de l'extrémité proximale (115) du dispositif d'insertion (110), comprenant une unité de serrage (10, 10a) permettant de serrer l'implant auto-expansible (105) dans le dispositif d'insertion (110), avec une extrémité proximale (12), qui est éloignée de l'extrémité distale (120) du dispositif d'insertion (110) dans l'état d'utilisation, et une extrémité distale (14) qui est orientée vers l'extrémité distale (120) du dispositif d'insertion (110) dans l'état d'utilisation, où l'unité de serrage (10, 10a) comprend un premier composant (16, 16a) et au moins un deuxième composant (18, 18a),
où le premier composant (16, 16a) est disposé à l'extrémité proximale (12) de l'unité de serrage (10, 10a) et le deuxième composant (18, 18a) est disposé à l'extrémité distale (14) de l'unité de serrage (10, 10a),
où le premier composant (16, 16a) et le deuxième composant (18, 18a) sont disposés fixés axialement sur la tige intérieure (52),
où l'implant auto-expansible (105) est serré entre la tige extérieure (54) et le deuxième composant (18, 18a) dans l'état serré,
où le premier composant (16, 16a) présente une zone d'enveloppe (38),
où la zone d'enveloppe (38), dans l'état serré, entoure une extrémité proximale (106) de l'implant auto-expansible (105) dans une direction circonférentielle (40) et peut ainsi créer un emboitement entre le premier composant (16, 16a) et l'implant auto-expansible (105), qui maintient l'implant auto-expansible (105) en position, **caractérisé en ce que** le premier composant (16, 16a) présente un matériau qui est plus dur que le matériau de l'au moins un deuxième composant (18, 18a), ce par quoi le premier composant (16, 16a) peut résister aux forces radiales du deuxième composant et/ou de l'implant.

2. Dispositif de libération selon la revendication 1, dans lequel, dans l'état serré, une friction entre l'au moins un deuxième composant (18, 18a) et l'implant (105) maintient celui-ci en position.

3. Dispositif de libération selon la revendication 1 ou la revendication 2, dans lequel le premier composant (16, 16a) est formé par une connexion femelle (20) et/ou l'au moins un deuxième composant (18, 18a) est formé par une connexion mâle (22).

4. Dispositif de libération selon l'une des revendications précédentes, dans lequel l'au moins un deuxième composant (18, 18a) présente un matériau avec un frottement statique élevé afin de maintenir en position l'implant auto-expansible (105) dans l'état serré.

5. Dispositif de libération selon la revendication 4, dans lequel le matériau est un polymère et en particulier, un matériau choisi dans le groupe constitué du polyamide, du polyester, d'un copolymère polyéther-amide, de silicone, de polyuréthane.

6. Dispositif de libération selon l'une des revendications précédentes, dans lequel le matériau du premier composant (16, 16a) est un matériau choisi dans le groupe constitué d'une matière plastique, notamment de polycarbonate, de poly éther éther cétone (PEEK), de perfluoro éthylène propylène, de polyéthylène haute densité, de poly tétrafluoro éthylène, de polyamide, d'un métal, d'une céramique, d'un caoutchouc dur ou d'un verre.

7. Dispositif de libération selon la revendication 6, dans lequel le matériau du premier composant (16, 16a) est un métal, de préférence, un acier inoxydable, notamment avec une surface structurée.

8. Dispositif de libération selon l'une des revendications précédentes, dans lequel le premier composant (16, 16a) et/ou l'au moins un deuxième composant (18, 18a) présentent un passage (24) pour la tige intérieure (52).

9. Dispositif de libération selon l'une des revendications précédentes, dans lequel le premier composant (16a) et/ou l'au moins un deuxième composant (18a) présentent un contour conique (28, 30) permettant l'interaction avec l'implant auto-expansible (105).

10. Système comprenant un dispositif de libération (100, 100a) selon l'une des revendications précédentes et un implant auto-expansible (105) serré dans le dispositif de libération (100, 100a).

11. Système selon la revendication 10, dans lequel un diamètre intérieur (Dᵢ₁₆) du premier composant (16, 16a) est au maximum de 0,2 mm plus large qu'un diamètre extérieur (Dₐ₁₀₆) d'une extrémité proximale (106) de l'implant (105) dans son état comprimé.

12. Système selon la revendication 10 ou la revendication 11, dans lequel le premier composant (16, 16a) présente au moins deux couches qui sont disposées l'une sur l'autre en direction radiale, où la couche extérieure radialement présente un matériau avec une faible friction par rapport à la tige extérieure (54) et la couche intérieure radialement présente un matériau avec une friction élevée par rapport à l'implant auto-expansible (105).

13. Dispositif d'insertion (110) permettant l'insertion d'un implant auto-expansible (105) médical comprenant un dispositif de libération (100, 100a) pour la libération de l'implant auto-expansible (105) médical, selon l'une des revendications 1 à 9.

14. Dispositif d'insertion selon la revendication 13, dans lequel un bouchon (125) est prévu qui limite le déplacement de l'implant auto-expansible (105) en direction d'une extrémité proximale (115) du dispositif d'insertion (110), où l'unité de serrage (10, 10a) présente le bouchon (125) et/ou le premier composant (16, 16a) de l'unité de serrage (10, 10a) est conçu en une seule pièce avec le bouchon (125).

15. Dispositif d'insertion selon la revendication 13 ou la revendication 14, dans lequel l'implant auto-expansible (105) est conçu dépourvu d'élément de fixation.

16. Procédé de serrage d'un implant auto-expansible (105) médical au moyen d'une unité de serrage (10, 10a) d'un dispositif de libération (100, 100a) selon l'une des revendications 1 à 9, dans un dispositif d'insertion (110) selon la revendication 13, chez lequel l'implant auto-expansible (105) est libéré par un déplacement relatif entre une tige intérieure (52) et une tige extérieure (54) et le retrait consécutif du dispositif d'insertion (110), où l'unité de serrage (10, 10a) comprend une extrémité proximale (12) qui est éloignée de l'extrémité distale (120) du dispositif d'insertion (110) dans l'état d'utilisation, et une extrémité distale (14) qui est orientée vers l'extrémité distale (120) du dispositif d'insertion (110) dans l'état d'utilisation, présentant au moins les étapes suivantes:
- la mise en place de l'implant auto-expansible (105) sur une tige intérieure (52) et au moins une partie (107) de l'implant auto-expansible (105) est disposée au dessus d'au moins un deuxième composant (18, 18a) de l'unité de serrage (10) de sorte qu'au moins une extrémité proximale (106) de l'implant auto-expansible (105) se trouve disposée de manière proximale par rapport au deuxième composant (18, 18a),
- le pliage d'au moins une extrémité proximale (106) de l'implant auto-expansible (105) de manière radiale en direction d'un axe intérieur (130) de l'implant auto-expansible (105);
- l'insertion au moins de l'extrémité proximale (106) repliée de l'implant auto-expansible (105) dans une zone d'enveloppe (38) du premier composant (16, 16a) de l'unité de serrage (10) de sorte qu'au moins une région (108) de l'implant auto-expansible (105) est adjacente radialement à au moins une zone (30) d'une surface extérieure (32, 32a) du deuxième composant (18, 18a);
- la mise en place de l'unité de serrage (10) avec l'au moins un implant auto-expansible (105) dans au moins une tige extérieure (54).
